(19) Europäisches Patentamt
European Patent Office
Office européen des brevets

(11) Veröffentlichungsnummer: **0 520 119 A1**

(12) # EUROPÄISCHE PATENTANMELDUNG

(21) Anmeldenummer: **91810460.5**

(51) Int. Cl.[5]: **A61K 31/195**, A61K 9/54

(22) Anmeldetag: **17.06.91**

(43) Veröffentlichungstag der Anmeldung:
**30.12.92 Patentblatt 92/53**

(84) Benannte Vertragsstaaten:
**AT BE CH DE DK ES FR GB GR IT LI LU NL SE**

(71) Anmelder: **SPIRIG AG PHARMAZEUTISCHE PRÄPARATE**
**Froschacker 434**
**CH-4622 Egerkingen(CH)**

(72) Erfinder: **Juch, Rolf-Dieter**
**Schänggelistrasse 32**
**CH-4612 Wangen b. Olten(CH)**

(74) Vertreter: **Braun, André**
**Murtengasse 5**
**CH-4051 Basel(CH)**

(54) **Neue orale Diclofenaczubereitung.**

(57) Die neue Diclofenaczubereitung mit gesteuerter Freisetzung liegt in Pelletform vor. Der auf Neutralpellets aufgetragene Wirkstoff wird mit einer Membranschicht überzogen, die nebst 45-55 Gew.-% eines wasserunlöslichen Polymers 6-13 Gew.-% mindestens eines wasserlöslichen und/oder wasserunlöslichen Porenbildners und 25-46 Gew.-% an Hilfsstoffen enthält. Die Porenbildner ermöglichen eine sehr gleichmässige Freisetzung von Diclofenac in der Art, dass eine zweimalige Applikation pro Tag ausreicht. Über die Membranschicht wird eine magensaftresistente Lackschicht aufgetragen.

EP 0 520 119 A1

Die Erfindung betrifft eine neue Diclofenaczubereitung mit gesteuerter Wirkstofffreisetzung, wie sie in den Patentansprüchen beschrieben wird.

Das nichtsteroide Diclofenac hat sich seit langem wegen seiner starken entzündungshemmenden und schmerzlindernden Wirkung in der Langzeittherapie von rheumatischen Erkrankungen bewährt.

Es ist jedoch allgemein bekannt, dass die Blutkonzentrations-Halbwertszeit von oralen Diclofenaczubereitungen mit einer bis zwei Stunden sehr kurz ist. Deswegen muss das Arzneimittel üblicherweise mindestens dreimal pro Tag verabreicht werden. Die häufige Einnahme des Arzneimittels bewirkt aber eine ungenügende Patienten compliance, zumal mit einer verschlechterten Mitwirkung der Patienten vor allem auch deswegen zu rechnen ist, weil diese häufig fortgeschrittenen Alters sind und täglich mehrere verschiedene Arzneimittel zu sich nehmen müssen. Dies erschwert jedoch eine klinische Kontrolle un den Therapieerfolg.

Problematisch können zudem die durch den raschen Wirkstoffanstieg im Blut bewirkten Nebeneffekte, wie z.B. Magen-Darmbeschwerden verschiedenster Art, sein. Da die therapeutische Behandlung einer rheumatischen Entzündung immer einen Kompromiss zwischen einer erfolgreichen Bekämpfung der Symptome, was einen ausreichenden Blutplasmaspiegel von Diclofenac erfordert, und einem Akzeptieren unerwünschter Nebenwirkungen, was bekanntlich durch überflüssig hohe Blutplasmawerte entscheidend beeinflusst wird, darstellt, hat man schon versucht, diese Nebeneffekte durch die Beeinflussung der Freisetzung von Diclofenac zu steuern.

Es ist bekannt, dass Diclofenac sich in der Synovialflüssigkeit mit Verzögerung aus dem Blutplasma anreichert. Die Austauschvorgänge Blutplasma-Synovialflüssigkeit verlaufen im Verhältnis zur Plasmakinetik langsam. Es ist deshalb nicht notwendig, einen hohen Blutplasmaspiegel über viele Stunden zu halten. Weil die Eliminationshalbwertszeit von nicht retardiertem Diclofenac in einem Bereich von 1-2 Stunden liegt, reicht jedoch in der Regel eine zweimalige orale Applikation am Tag nicht aus. So erwähnt Fenner in Tempo Medical (APV-Kurs über Antirheumatika, Nürnberg 1983),
dass selbst retardierte Formen von Diclofenac nur bedingt geeignet sind, den durch die kurzen Blutplasmaeliminationshalbwertszeiten bedingten Plasmaspiegelverlauf so zu beeinflussen, dass auch noch nach 8 bis 10 Stunden eine nennenswerte Steuerung des Blutplasmaspiegels erfolgen kann, weil die Retardierung nach diesem Zeitraum zu einer starken Abflachung der Blutplasmaspiegelkurve führt.

Dem Fachmann stellt sich bei der Entwicklung neuer galenischer Verabreichungsformen deshalb immer das Problem, den Bluntplasmaspiegel von Diclofenac einerseits so hoch zu halten, dass die Wirkung auch über Nacht anhält und so beispielsweise Symptome wie Morgensteifigkeit vermieden werden können, und andererseits die Wirkung von Diclofenac schon kurz nach der Einnahme eintritt.

Zur Erreichung dieses Ziels wird häufig empfohlen, die Einnahme eines magensaftresistenten Dragées mit der Verabreichung eines Suppositoriums oder Retard-Dragées vor dem Schlafengehen zu kombinieren. Dies kann für den Patienten zu folgenschweren Verwechslungen mit erhöhten Nebenwirkungen oder mangelhafter kurzfristiger Schmerzlinderung führen.

Um dies zu vermeiden, hat man versucht, beide Wirkungsarten von Diclofenac in einer einzigen Verabreichungsform zu kombinieren.

In der JP 61/44811 (Veröffentlichungsdatum 4. März 1986) wird eine Kombination eines initial-freisetzenden Granulatgemisches mit verzögert freisetzendem Wirkstoffanteil beansprucht. Da der Wirkstoff im initial-freisetzenden Granulatanteil schon im Magen freigesetzt wird, ist wegen der schleimhautreizenden resp. -schädigenden Wirkung von Diclofenac mit nachteiligen Nebenwirkungen zu rechnen.

Der Diclofenac verzögert freisetzende Granulatanteil ist mit einer Hülle enthaltend Methacrylsäure-Methylmethacrylat-Copolymer beschichtet, die sich in Wasser bei einem pH von 6-7 löst. Diese Hülle neigt bei der Beschichtung und Trocknung zum Bruch. Die Bruchstücke weisen eine Tendenz zum Agglomerieren auf. Dadurch wird einerseits die Ausbeute an Partikeln der gewünschten Grösse vermindert und andererseits die verzögert-freisetzende Wirkung dieser Partikel aufgehoben.

Ein weiterer Entwicklungsschritt bei Diclofenacformulierungen besteht darin, obige Nachteile zu beheben. So wird in EP 348 808 ein pelletiertes orales Diclofenacpräparat beschrieben, dessen Wirkstoff zu einem Teil in retardiert freisetzender und zu einem andern Teil in magensaftresistenter Form vorliegt. Erreicht wird dieses Ziel durch Umhüllung eines Teils der Pellets mit einer für Diclofenac retardiert durchlässigen Diffusionsmembran und eines andern Teils der Pellets mit einer magensaftresistenten Membran. Die Diffusionsmembran besteht dabei aus mindestens einer Schicht eines Acrylharzes. Da Diclofenac Säurecharakter mit einem pKa-Wert von ca. 4-5 aufweist, nimmt dessen Grundlöslichkeit im wässrigen Medium oberhalb von diesem Wert wesentlich zu. Damit nimmt in der Regel auch die Wechselwirkung mit den Polymeren, die für die magensaftresistente Umhüllung von Pellets verwendet werden, zu. Bei gleichzeitiger Diclofenaceinnahme und Nahrungsaufnahme kann dann bei einer dadurch bedingten Erhöhung des pH-Werts bis ca. 5 im Magen der Wirkstoff durchaus schon freigesetzt werden.

Um dies zu verhindern, wird eine zweischichtige magensaftresistente Membran aus Celluloseether mit unterschiedlichem Substitutionsgrad, die mit Phthalsäureanhydrid verestert ist, vorgeschlagen, wobei in deren inneren Schicht als Hilfsstoff eine wasserunlösliche organische Säure eingearbeitet wird.

Ziel der in der EP 383 967 beschriebenen Erfindung ist es, ein pelletiertes orales Diclofenacpräparat mit verlängerter Wirkung und verbesserter magensaftresistenter Hülle bereitzustellen, so dass die Partikel nicht mehr agglomerieren.

Gelöst wird das Problem durch ein Präparat mit einem Anteil an schnell-freisetzendem Diclofenac und einem Teil magensaftresistent umhüllter Pellets. Die Hülle besteht aus 100 Teilen Methacrylsäure-Methylmetacrylat-Copolymer, 3-40 Teilen Glycerinfettsäureester und 1-150 Teilen Talcum.

Auch in der DE-OS 39 15 150 wird ein langwirkendes Diclofenacpräparat beansprucht, das eine Komponente mit rascher Wirkstofffreigabe und eine mit verzögerter Wirkstofffreigabe umfasst. Dabei wird die mit verzögerter Freigabe enthaltende Diclofenac-Komponente mit einer organischen Säure vermischt und das Gemisch mit einer üblichen Hülle versehen.

Bei den vorerwähnten Kombinationspräparaten wird jeweils ein schnell-freisetzendes und verzögert-freisetzendes Diclofenac miteinander gemischt und dann meist zu Tabletten gepresst oder in Kapseln gefüllt. Eine quantitativ exakte Mischung herzustellen, ist jedoch kompliziert. Das Abfüllen zweier Komponenten in eine Kapsel ist aber aus einer Mischung wegen möglicher Inhomogenitäten problematisch; eine getrennte Abfüllung zweier Komponenten ist technisch aufwendiger und bei derart kleinen Füllmengen wegen der erforderlichen Dosierungsgenauigkeit nicht ratsam.

Da schon kleine Schwankungen in der quantitativen Zusammensetzung oder nicht homogenes Vermischen der Komponenten eines Diclofenacpräparats bedeutende Schwankungen im Blutplasmawert hervorrufen können, wäre es von grossem Vorteil, ein Diclofenacpräparat mit gesteuerter Wirkstofffreisetzung, das nur aus einer einzigen Komponente besteht, bereitzustellen.

Aufgabe der vorliegenden Erfindung ist es deshalb, eine Diclofenac-haltige orale Zubereitung mit gesteuerter Wirkstofffreisetzung zur Verfügung zu stellen, bei dessen Einnahme möglichst sofort eine Schmerzlinderung eintritt und diese über einen längeren Zeitraum von ca. 12 Stunden erhalten bleibt. In dieser Schrift bedeutet Diclofenac die freie Säure und deren Salze, insbesondere Diclofenac-Na.

Weitere Aufgaben der Erfindung bestehen darin, ein Höchstmass an Reproduzierbarkeit für die Freisetzung des Wirkstoffs zu erzielen, keine organischen Lösungsmittel beim Aufbau des Präparats zu verwenden, um das Verbleiben von Restlösemittel im Endprodukt zu vermeiden, und eine mehrjährige Stabilität in der Freisetzungscharakteristik bei normaler Lagerung der Zubereitung zu gewährleisten.

Gelöst wurden diese Aufgaben erfindungsgemäss durch eine Diclofenac-haltige pelletierte orale Arzneimittelzubereitung mit gesteuerter Wirkstofffreisetzung, die eine auf einem Neutralpellet aufgetragene Wirkstoffschicht, eine 10-20 Gew.-% der Endpellets betragende innere Membranschicht, wobei 45-55 Gew.-% der Membranschicht aus mindestens einem wasserunlöslichen Polymer, 25-55 Gew.-% aus mindestens einem Porenbildner und bis zu 30 Gew.-% aus Hilfsstoffen besteht, und eine äussere magensaftresistente Lackschicht enthält.

Bevorzugterweise wird die erfindungsgemässe Arzneimittelzubereitung in Pelletform in eine Kapsel, besonders bevorzugt in eine Hartgelatinekapsel, eingeschlossen.

Durch die erfindungsgemässe multiple-unit Arzneimittelform werden die bisher vorhandenen Probleme bei der Herstellung und Verabreichung von Diclofenacpräparaten weitgehend gelöst.

Die Wirkstoffschicht enthält den Wirkstoff und Hilfsstoffe, die erfindungsgemäss auf handelsübliche Neutralpellets (Nonpareils) aufgetragen werden.

Geeignete Korngrössen der Neutralpellets weisen einen Durchmesser von 0,5 mm bis 1,2 mm, vorzugsweise 0,6 mm bis 0,85 mm mit weniger als 5 Gew.-% Über- resp. Untergrössen, auf. Der Aufbau mit Diclofenac erfolgt dann mit üblichen Auftragstechniken, z.B. in rotierenden Kesseln, vorzugsweise aber in Wirbelschichtsprühanlagen, die vorzugsweise mit einem Wurster-Einsatz oder Rotorprozessor-Einsatz bestückt sind.

Die Zugabe von Diclofenac erfolgt vorzugsweise in einer Suspension in Wasser, die als Hilfsstoffe vorzugsweise Bindemittel, wie Povidon oder Cellulosederivate, wie z.B. Hydroxypropylcellulose oder Hydroxypropylmethylcellulose, Weichmacher, wie Macrogol, und Gleit- resp. Trennmittel, wie Talcum, Mg-Stearat, Syloid oder Aerosil, und gegebenenfalls eine Silicon-Antischaumemulsion enthält. Der Suspension kann als "Indikator" für die Überprüfung der Magensaftresistenz gemäss CH-Patent 656 310 z.B. Nicotinamid zugesetzt werden.

Als besonders bevorzugte Hilfsstoffe wird ein Gemisch, das Povidone K 30, Macrogol 400, Talcum und als Indikator gegebenenfalls eine Silicon-Antischaumemulsion und/oder Nicotinamid enthält, verwendet.

Die Diclofenac enthaltende Suspension wird vorzugsweise durch Aufsprühen auf die Neutralpellets aufgetragen. Die derart mit der Wirkstoffschicht beladenen Neutralpellets werden bis auf eine Restfeuchte von 0,1-4,0 Gew.-%, vorzugsweise 0,4-1,0 Gew.-%, jeweils bezogen auf die unbeschichteten Pellets, getrocknet. Der Aufbau der Wirkstoffschicht ist beendet, wenn die Neutralpellets um 110-160 Gew.-%, vorzugsweise 130-140 Gew.-%, schwerer geworden sind. Solcherweise werden vorzugsweise Pellets mit einem Wirkstoffanteil von 20-45 Gew.-% und 5-15 Gew.-% Hilfsstoffe, bezogen auf die Endpellets (als Endpellets werden die erfindungsgemäss zweifach beschichteten Pellets bezeichnet) , hergestellt.

Durch Siebung der Pellets kann man die für die Reproduzierbarkeit der Freisetzung geeignete Pelletgrösse im Bereich von 0,6 mm bis 1,4 mm, vorzugsweise 0,8 mm bis 1,25 mm, aussortieren.

Die angestrebte Wirkstofffreisetzung wird insbesondere bestimmt durch den Aufbau der inneren Membranschicht und der äusseren Lackschicht.

Die innere Membranschicht steuert die Diffusion von Diclofenac aus dem Innern des Pellets in die umgebende Flüssigkeit. Zur Steuerung der Diffusionscharakteristik und zur Gewährleistung der gewünschten Freisetzung von Diclofenac ist es notwendig, dass diese Schicht im Laufe der Diffusion und Wirkstoffverarmung des Pellets nicht platzt oder sich auflöst, sondern nur schrumpft, den Wirkstoff aber gleichwohl in der gewünschten Menge freisetzt.

Erreicht wird dieses Ziel durch die Applikation eines Gemisches, das mindestens ein geeignetes wasserunlösliches Polymer, wie z.B. wasserunlösliche Acrylharze, z.B. Acrylsäure-Methacrylestercopolymere, wie sie unter der Bezeichnung Eudragit RL 30 D, Eudragit RS 30 D oder Eudragit NE 30 D von Röhm, Darmstadt, Deutschland, vertrieben werden, Surelease oder Surelease XM in einer Menge von 45-55 Gew.-%, mindestens einen geeigneten wasserlöslichen oder wasserunlöslichen Porenbildner oder ein Gemisch davon in einer Menge von 6-13 Gew.-%, und Hilfsstoffe, wie z.B. Weichmacher, wie Triacetin oder Diethylphthalat, oder Gleitmittel, wie z.B. Talcum, Mg-stearat, Syloid oder Aerosil, und gegebenenfalls Antischaumemulsionen, vorzugsweise eine Silicon-Antischaumemulsion, in einer Menge von 25-45 Gew.-%, jeweils bezogen auf die Membranschicht, enthält.

Als wasserunlösliche Porenbildner können z.B. Kaolin, Calciumcarbonat, Calciumhydrogenphosphat, Magnesiumoxid, mikrokristalline Cellulose, Titandioxid oder Eisenoxid verwendet werden.

Als wasserlösliche Porenbildner können z.B. Povidone K 30, Polyvinylalkohol, Cellulosederivate, wie Hydroxypropylcellulose, Hydroxypropylmethylcellulose, Methylcellulose oder Natriumcarboxymethylcellulose, Saccharose, Xylit, Sorbit, Mannit, Maltose, Xylose, Glucose, Kaliumchlorid, Natriumchlorid, Polysorbat 80, Polyethylenglykol, Propylenglykol oder Natriumcitrat verwendet werden.

Es kann dabei ein einzelner Porenbildner, aber auch ein Gemisch von wasserunlöslichen oder wasserlöslichen Porenbildnern oder ein Gemisch von wasserlöslichen und wasserunlöslichen Porenbildnern verwendet werden.

Bevorzugt wird ein Porenbildnergemisch von Povidone K 30, Polysorbat 80 und Eisenoxid rot verwendet.

Die Membranschicht kann man auf verschiedene an sich bekannte Arten auftragen. Günstige Resultate werden besonders durch Aufsprühen einer wässrigen Suspension der Membranschichtbildner auf die vorgeformten Pellets in vorzugsweise einer Wirbelschichtsprühanlage erreicht. Besonders bevorzugt enthält die Wirbelschichtsprühanlage einen Wurster-Einsatz oder einen Rotorprozessor-Einsatz.

Das Gewicht der Membranschicht beträgt vorzugsweise 10-20 Gew.-% der Endpellets. Bevorzugt liegt der Anteil bei etwa 15 Gew.-%, wobei eine Änderung der Menge die Freisetzungscharakteristik wesentlich beeinflusst.

Eine bevorzugte Ausführungsform der aufgetragenen Membranschicht enthält in Gew.-% der Membranschicht z.B.:

| | | |
|---|---|---|
| Eudragit NE 30 D | 45 % bis 55 % | insbesondere 53,5 % |
| Povidone K 30 | 5 % bis 10 % | insbesondere 8,8 % |
| Polysorbat 80 | 0,5 % bis 1,5 % | insbesondere 1,3 % |
| Eisenoxidrot | 0,5 % bis 1,5 % | insbesondere 0,8 % |
| Talcum | 15 % bis 30 % | insbesondere 22,1 % |
| Mg-stearat | 10 % bis 15 % | insbesondere 13,3 % |
| Antischaum-Emulsion SE2 | 0,01% bis 0,1 % | insbesondere 0,05% |

Eudragit NE 30 D wird dabei als schichtbildendes wasserunlösliches Copolymer eingesetzt. Die Porenbildner haben die Aufgabe, sich im Darmtrakt aus der Membranschicht herauszulösen oder herauszubrechen, damit die umgebende Flüssigkeit in das Pellet hineindiffundieren und dort Diclofenac auflösen kann. Das gelöste Diclofenac kann dann gemäss der beabsichtigten Freisetzungscharakteristik durch die Poren der Membranschicht in den Magen-/Darmtrakt entlassen werden. Dank dem vordefinierten Aufbau der Membranschicht wird praktisch der gesamte vorhandene Wirkstoff vom Körper resorbiert.

Ein weiterer Vorteil der erfindungsgemässen Membranschicht besteht darin, dass ein Dosierungsintervall der Zubereitung von 12 Stunden genügt, ohne dass es zur schädlichen Kumulation von Wirkstoffkonzentration im Blutplasma kommt und trotzdem eine relativ hohe Anfangskonzentration erreicht werden kann, so dass damit ein frühes Ausfluten des Wirkstoffs in die Synovialflüssigkeit gefördert wird.

In einem weiteren Vorgang wird eine magensaftresistente Lackschicht aufgetragen, die sich oberhalb eines pH-Wertes von 5,5 auflöst. Vorzugsweise wird diese Schicht, die bevorzugt 5-15 Gew.-% der Endpellets, und besonders bevorzugt 10 Gew.-%, ausmacht, aufgesprüht. Die magensaftresistente Lackschicht selbst enthält 60-90 % mindestens eines säureunlöslichen Polymers und 10-40 % Hilfsstoffe.

Als säureunlösliche Polymere kommen z.B. Methacrylsäure-Methacrylsäureester-Copolymere oder Methacrylsäure-Acrylsäureester-Copolymere, wie z.B. Eudragit L, Eudragit S, Eudragit RL 30 D und/oder Eudragit RS 30 D, oder Aquateric oder Duodcel in Frage, und vorzugsweise Eudragit L 30 D.

Als Hilfsstoffe kommen Weichmacher, wie z.B. Diethylphthalat, Triacetin, Dibutylsebacat, Diethyladipat, Polyethylenglykol oder Tributylcitrat, und Gleit- resp. Trennmittel, wie z.B. Talcum, Syloid, oder Farbpigmente, wie z.B. Eisenoxid oder Titandioxid, und gegebenenfalls eine Silicon-Antischaumemulsion, wie sie z.B. unter der Bezeichnung SE 2 von der Firma Wacker angeboten wird, in Frage.

Eine bevorzugte Form der magensaftresistenten Lackschicht enthält 70-80 Gew.-% Eudragit L 30 D, 10-20 Gew.-% Diethylphthalat, 5-15 Gew.-% Talcum und gegebenenfalls 0,1 -0,5 Gew.-% (Trockenanteil 0,02-0,1 Gew.-%), jeweils bezogen auf die Lackschicht, eines Silicon-Antischaummittels.

Die magensaftresistente Lackschicht wird bis zu einem Anteil von 5-15 Gew.-% der Endpellets, vorzugsweise ca. 10 Gew.-%, aufgetragen. Falls die Schicht die in USP XXII, S. 1580, festgelegten Kriterien für Magensaftresistenz nicht erfüllt, kann die Lackschicht durch zusätzliches Auftragen weiter verstärkt werden. Die erfindungsgemässe wässrige Suspension für das Auftragen der magensaftresistenten Lackschicht lässt sich ausgezeichnet verarbeiten, wobei das Auftragen an sich auf herkömmliche Art erfolgen kann. Vorzugsweise erfolgt das Aufsprühen der magensaftresistenten Lackschichtbildner jedoch in einer wässrigen Suspension in einer Wirbelschichtsprühanlage mit Wurster-Einsatz oder Rotorprozessor-Einsatz.

Die mit der magensaftresistenten Lackschicht umhüllten Pellets werden zur Entfernung von Agglomeraten und eventuellen Fragmenten gesiebt und klassiert. Die Fraktion zwischen 0,6 mm und 1,4 mm, vorzugsweise 0,8 mm bis 1,25 mm, eignet sich vorzüglich für die Freisetzung von Diclofenac mit den in-vitro-Parametern (USP XXII, S. 1580):

> 15 % nach 1 Stunde
> 30 % nach 2 Stunden
> 50 % nach 3 Stunden
> 70 % nach 4 Stunden
> 80 % nach 5 Stunden.

Die erfindungsgemässe Lackschicht weist eine gute Langzeitstabilität auf, so dass auch noch nach 2 Jahren Lagerung die Freisetzungskinematik in-vitro im wesentlichen erhalten bleibt (vgl. Fig.1).

Da die einzelnen Pelletschichten einerseits für sich allein schon mengenmässig in gewissen Grenzen variabel aufgebaut sein können, der mengenmässig variable Aufbau andererseits aber auch zwischen den einzelnen Schichten möglich ist, wurden die Prozentangaben teils auf die einzelnen Schichten und teils auf die Endpellets bezogen.

Im folgenden soll deshalb die art- und mengenmässige Zusammensetzung bevorzugter Endpellets in Gew.-% wiedergegeben werden:

| | | | |
|---|---|---|---|
| a) Neutralpellet | 20 % bis 45 % | insbesondere | 31,0 % |
| b) Wirkstoffschicht+): | | | |
| Diclofenac Na | 20 % bis 45 % | insbesondere | 31,0 % |
| Hilfsstoffe | 5 % bis 15 % | insbesondere | *) |
| c) Membranschicht+): | | | |
| Eudragit NE 30 D (Trockenanteil) | 6 % bis 14 % | insbesondere | 8,0 % |
| Povidone K 30, | 0,05% bis 4 % | insbesondere | 1,5 % |
| Polysorbat 80, | 0,05% bis 1 % | insbesondere | 0,2 % |
| Eisenoxid rot | 0,05% bis 3 % | insbesondere | 0,15 % |
| Hilfsstoffe vorzugsweise | | | |
| Talcum | 1 % bis 6 % | insbesondere | 3,5 % |
| Mg-stearat | 0,5 % bis 3 % | insbesondere | 2,0 % |
| d) Lackschicht+): | | | |
| Eudragit L 30 D (Trockenanteil) | 5 % bis 12 % | insbesondere | 7,5 % |
| Diethylphthalat, Talcum | 1 % bis 4 % | insbesondere | **) |

+) gegebenenfalls eine Silicon-Antischaumemulsion enthaltend

*) Povidone K 30 6 %
Macrogol 400 1 %
Talcum 2 %
gegebenenfalls Nicotinamid 3 %

**) Diethylphthalat 1,5 %
Talcum 1 %

Vorzugsweise werden die Pellets in Kapseln, besonders bevorzugt in Hartgelatinekapseln, abgefüllt. Therapeutisch ist eine Füllmenge von 50 mg bis 150 mg Diclofenac pro Kapsel, bevorzugt 75 mg (entspricht ca. 155 mg bis 165 mg an Pellets), erforderlich.

Mit der bevorzugten Dosierung von Diclofenac und der unter in-vitro-Bedingungen zu standardisierenden und kontrollierbaren Freisetzungscharakteristik erfolgt nahezu vollständige Resorption des Wirkstoffs im Körper. Die maximalen Blutplasmawerte liegen im Bereich von 300 ng/ml und das Wirkstoffplateau wird wegen der retardierten Wirkstofffreigabe während mehrerer Stunden im Serum aufrechterhalten.

Selbst wenn während längerer Zeit alle 12 Stunden eine erfindungsgemässe Kapsel mit z.B. 75 mg Diclofenac verabreicht wird, kann keine Kumulation der Blutplasmawerte beobachtet werden, weil die Maximalkonzentrationen jeweils innerhalb eines sehr engen Zeitraums erreicht werden. Auch ist die Streuung der gemessenen Blutplasmawerte offensichtlich geringer als nach Verabreichung von Zubereitungen nach dem Stand der Technik.

Ein weiterer Vorteil der Erfindung besteht darin, dass es für die Freisetzung von Diclofenac - im Gegensatz zum Stand der Technik - nicht darauf ankommt, ob die Zubereitung einer zweistündigen in-vitro Magensaft-Vorbehandlung unterworfen worden ist. Dies wird aus Fig.2 und Fig.3 ersichtlich, wo die Mittelwerte der Freisetzung im künstlichen Darmsaft von 2 verschiedenen Herstellungschargen einer 75 mg enthaltenden erfindungsgemässen Diclofenaczubereitung einmal mit (Fig.2, Probe A1 und Probe B1) und einmal ohne (Fig.3, Probe A2 und Probe B2) zweistündiger Magensaftvorbehandlung gemessen wurden.

Dieser in-vitro-Versuch wurde an die US-Paddle-Methodik angelehnt und mit dem Gerät SOTAX AT6 nach USP XXI durchgeführt. Die mittlere Dissolutionszeit (sie repräsentiert das Zeitintervall bis zur Freisetzung von 63,2 % Wirkstoff) lag dabei unabhängig von der Vorbehandlung bei ca. 2 Stunden.

Die mittlere Verweilzeit (MRT = mean residence time) des Wirkstoffs beträgt in einer bevorzugten Ausführungsform ca. 5,5 Stunden.

Die geringen Standard-Abweichungen und der fehlende Einfluss einer zweistündigen Vorbehandlung mit Magensaft sind ein Beweis für das regelmässige Verhalten der Freisetzung von Diclofenac aus einer erfindungsgemässen Arzneimittelzubereitung unter sehr verschiedenen Bedingungen. Dies führt in vivo zu einer verringerten Streuung der Blutplasma-Konzentrationen, was für die bekanntermassen an sich schon sehr schwankungsreichen Blutplasma-Konzentrationsverläufe von Diclofenac von ausschlaggebender Bedeutung ist.

Ein besonderer Vorteil der erfindungsgemässen Zubereitung besteht deshalb darin, dass mit der täglichen Verabreichung einer erfindungsgemässen 2 x 75 mg Zubereitung vergleichbare Resultate wie mit einer 3 x 50 mg Standard-Dosierung und bessere Resultate als mit 2 x 100 mg Retarddosierungen gemäss dem Stand der Technik erzielt werden können.

Die Herstellung der beschichteten Pellets kann nach herkömmlichen Verfahren erfolgen, vorzugsweise wird dazu jedoch das Wirbelschichtsprühverfahren benützt. Durch Anwendung dieses Verfahrens und Verwendung der beschriebenen Hilfsmittel brauchen keine organischen Lösungsmittel verwendet werden, so dass auch keine Restlösemittel im Endprodukt anfallen können. Zudem kann durch das unkomplizierte und ökonomische Herstellungsverfahren ein Höchstmass an Reproduzierbarkeit der Freisetzung erzielt, und auf Grund der einheitlich aufgebauten Pellets eine sichere Dosierung bei der Abfüllung in Kapseln sichergestellt werden.

Im folgenden wird die Erfindung anhand von Beispielen näher erläutert:

Beispiel 1

Herstellungsformel für 256 kg Pellets

| | |
|---|---|
| Neutralpellets | 80 kg |

Wirkstoffschicht (Suspension A)

| | |
|---|---|
| Aqua purificata | 240 kg |
| Silicon-Antischaumemulsion mit einem Trockenanteil von 0.13 kg | 0,65 kg |
| Diclofenac Natrium | 80 kg |
| Nicotinamid | 8 kg |
| Povidone 30 | 16 kg |
| Macrogol 400 | 2,4 kg |
| Talcum | 4,8 kg |

Membranschicht (Suspension B)

| | |
|---|---|
| Aqua purificata | 53 kg |
| Eudragit NE 30 D mit einem Trockenanteil von 21 kg | 70 kg |
| Polysorbat 80 | 0,5 kg |
| Povidone mit K-Value 30 | 3,5 kg |
| Eisenoxid rot E 172 (CI 77491) | 0,35 kg |
| Talcum | 9 kg |
| Magnesium stearat | 5 kg |
| Silicon-Antischaumemulsion mit einem Trockenanteil von 0.02 kg | 0,1 kg |

Lackschicht (Suspension C)

| | |
|---|---|
| Aqua purificata | 53 kg |
| Eudragit L 30 D mit einem Trockenanteil von 19.2 kg | 64 kg |
| Diethylphthaltat | 3,8 kg |
| Talcum | 2,7 kg |
| Silicon-Antischaumemulsion mit einem Trockenanteil von 0.02 kg | 0,1 kg |

In einer Wirbelschichtsprühanlage mit Wurster-Einsatz werden die Neutralpellets vorgelegt und mit der Suspension A besprüht. Die Bedingungen sind abhängig vom Gerätetyp und vom Fortschritt des Sprühprozesses.

Anschliessend an die Aufbringung der Wirkstsoffschicht in einer Wirbelschichtsprühanlage mit Wurster-Einsatz werden die Pellets mit Suspension B und mit Suspension C besprüht.

Bei Suspension A beträgt die Ablufttemperatur max. 50° C, bei Suspension B und C max. 40° C. Die rel. Feuchtigkeit (hygro-control aw2 Stankowitz, Diepholz, Deutschland) beträgt 25 bis 40 Gew.-%. Sieben und Klassieren erfolgt über Netzsiebe 0,8 mm und 1,4 mm. Korngrössenverteilung (Siebanalyse) liegt typisch im Bereich: < 0,2 Gew.-% < 0,8 mm und < 0,2 Gew.-% > 1,4 mm. Die Ausbeute liegt bei 90-98 Gew.-%.

Beispiel 2

Herstellungsformel für 205 kg Pellets

| | |
|---|---|
| Neutralpellets | 80 kg |

Wirkstoffschicht (Suspension A)

| | |
|---|---|
| Aqua purificata | 209 kg |
| Diclofenac Natrium | 80 kg |
| Hydroxypropylmethylcellulose | 8 kg |
| Syloid 244 FP | 2 kg |
| Silicon-Antischaumemulsion mit einem Trockenanteil von 0,1 kg | 0.5 kg |

Membranschicht (Suspension B)

| | |
|---|---|
| Aqua purificata | 30 kg |
| Eudragit NE 30 D mit einem Trockenanteil von 9,0 kg | 30 kg |
| Talcum | 8 kg |
| Silicon Antischaumemulsion mit einem Trockenanteil von 0,02 kg | 0.1 kg |

Lackschicht (Suspension C)

| | |
|---|---|
| Aqua purificata | 26 kg |
| Eudragit L 30 D mit einem Trockenanteil von 12 kg | 40 kg |
| Dibutylsebacat | 2,5 kg |
| Talcum | 3,5 kg |
| Silicon Antischaumemulsion mit einem Trockenanteil von 0,02 kg | 0,1 kg |

Das Herstellungsverfahren wird analog Bsp. 1 durchgeführt.

Beispiel 3

Herstellungsformel für 219 kg Pellets

| | |
|---|---|
| Neutralpellets | 80 kg |

Wirkstoffschicht (Suspension A)

| | |
|---|---|
| Aqua purificata | 230 kg |
| Diclofenac Natrium | 80 kg |
| Hydroxy propyl cellulose | 10 kg |
| Magnesiumstearat | 1 kg |
| Nicotinamid | 8 kg |
| Silicon Antischaumemulsion mit einem Trockenanteil von 0,02 kg | 0,1 kg |

Membranschicht (Suspension B)

| | |
|---|---|
| Aqua purificata | 26 kg |
| Eudragit RL 30 D mit einem Trockenanteil von 3,0 kg | 10 kg |
| Eudragit RS 30 D mit einem Trockenanteil von 9,0 kg | 30 kg |
| Triacetin | 2.5 kg |
| Talcum | 3.5 kg |
| Silicon-Antischaumemulsion mit einem Trockenanteil von 0,01 kg | 0,05 kg |

Lackschicht (Suspension C)

| | |
|---|---|
| Aqua purificata | 78 kg |
| Aquateric | 15 kg |
| Diethylphthalat | 5 kg |
| Polysorbat 80 | 0,2 kg |
| Titandioxid | 2 kg |

Das Herstellungsverfahren wird analog Bsp. 1 durchgeführt.

Beispiel 4

Herstellungsformel für 134 kg Pellets.

| | |
|---|---|
| Neutralpellets | 40,0 kg |

Wirkstoffschicht (Suspension A)

| | |
|---|---|
| Aqua purificata | 90,0 kg |
| Diclofenac Natrium | 40,0 kg |
| Nicotinamid | 4,0 kg |
| Povidone K 30 | 1,0 kg |
| Talcum | 2,0 kg |
| Silicon Antischaumemulsion mit Trockenanteil von 0,06 kg | 0,3 kg |

Membranschicht (Suspension B)

| | |
|---|---|
| Aqua purificata | 38,0 kg |
| Eudragit NE 30 D mit Trockenanteil von 15,3 kg | 51,0 kg |
| Eisenoxid rot | 0,25 kg |
| Polysorbat 80 | 0,35 kg |
| Talcum | 6,5 kg |
| Magnesiumstearat | 3,5 kg |
| Hydroxypropylmethylcellulose | 2,5 kg |
| Silicon Antischaumemulsion mit Trockenanteil von 0,02 kg | 0,1 kg |

Lackschicht (Suspension C)

| | |
|---|---|
| Aqua purificata | 38,0 kg |
| Eudragit L 30 D mit Trockenanteil von 13,8 kg | 46,0 kg |
| Diethylphthalat | 2,8 kg |
| Talcum | 2,0 kg |
| Silicon Antischaumemulsion mit Trockenanteil von 0,02 kg | 0,1 kg |

Das Herstellungsverfahren wird analog Beispiel 1 durchgeführt mit den folgenden Ausnahmen:

In einer Wirbelschichtsprühanlage mit Rotorprozessor-Einsatz werden die Neutralpellets vorgelegt und mit Suspension A 5 Min. mit einer Sprührate von 200 g/Min besprüht. Anschliessend wird der Innenzylinder auf eine Spalthöhe von 10 mm geöffnet. Die Prozessluft wird auf 1000 bis 1500 $m^3$/Std eingestellt. Mit

fortschreitendem Sprühprozess kann die Sprührate kontinuierlich bis auf 400-500 g/Min gesteigert werden.

Anschliessend an die Aufbringung der Wirkstoffschicht in einem Rotorprozessor werden die Pellets mit Suspension B und C besprüht.

Die Ablufttemperatur liegt bei allen 3 Sprühsuspensionen bei 30-40 °C. Die Scheibe dreht mit 500-900 U/Min.

**Patentansprüche**

1. Eine Diclofenac oder eines seiner Salze enthaltende pelletierte orale Arzneimittelzubereitung mit gesteuerter Wirkstofffreisetzung, gekennzeichnet durch eine auf einem Neutralpellet aufgebaute Wirkstoffschicht, eine innere Membranschicht, die 10-20 Gew.-% der Endpellets ausmacht und 45-55 Gew.-% eines wasserunlöslichen Polymers, 6-13 Gew.-% eines Porenbildners und 25-46 Gew.% Hilfsstoffe enthält, und eine äussere magensaftresistente Lackschicht.

2. Arzneimittelzubereitung nach Patentanspruch 1, dadurch gekennzeichnet, dass die Wirkstoffschicht 20-45 Gew.-% Wirkstoff und 5-15 Gew.-% Hilfsstoffe, bezogen auf die Endpellets, enthält.

3. Arzneimittelzubereitung nach Patentanspruch 1 oder 2, dadurch gekennzeichnet, dass die Wirkstoffschicht als Hilfsstoffe Bindemittel, vorzugsweise Povidone K 30, oder ein Cellulosederivat, wie Hydroxypropylcellulose oder Hydroxypropylmethylcellulose, Gleit- resp. Trennmittel, wie Mg-stearat, Aerosil und/oder Syloid 244 FP oder vorzugsweise Talcum, gegebenenfalls einen Weichmacher, vorzugsweise Macrogol 400, gegebenenfalls eine Silicon-Antischaumemulsion und gegebenenfalls einen Indikator, wie Nicotinamid, enthält.

4. Arzneimittelzubereitung nach Patentanspruch 1, dadurch gekennzeichnet, dass die Membranschicht als wasserunlösliches Polymer Eudragit RL 30 D, Eudragit RS 30 D, Eudragit NE 30 D, Surelease und /oder Surelease XM enthält.

5. Arzneimittelzubereitung nach einem der Patentansprüche 1 oder 4, dadurch gekennzeichnet, dass die Membranschicht als Porenbildner wasserunlösliche Stoffe, oder wasserlösliche Stoffe, oder ein Gemisch aus wasserlöslichen und wasserunlöslichen Stoffen enthält.

6. Arzneimittelzubereitung nach Patentanspruch 5, dadurch gekennzeichnet, dass als wasserunlösliche Stoffe Kaolin, Calciumcarbonat, Calciumhydrogenphosphat, Magnesiumoxid, mikrokristalline Cellulose, Titandioxid und/oder Eisenoxid verwendet werden.

7. Arzneimittelzubereitung nach Patentanspruch 5, dadurch gekennzeichnet, dass als wasserlösliche Stoffe Povidone, Polyvinylalkohol, Cellulosederivate, wie Hydroxypropylcellulose, Hydroxypropylmethylcellulose, Methylcellulose oder Natriumcarboxymethylcellulose, Polysorbat 80, Saccharose, Xylit, Sorbit, Mannit, Maltose, Xylose, Glucose, Kaliumchlorid, Natriumchlorid, Polyethylenglykol, Propylenglykol und/oder Natriumcitrat verwendet werden.

8. Arzneimittelzubereitung nach einem der Patentansprüche 1 oder 4-7, dadurch gekennzeichnet, dass die Membranschicht als Hilfsstoffe Weichmacher, wie Triacetin oder Diethylphthalat, und/oder Gleitmittel, wie Talcum, Mg-stearat, Syloid oder Aerosil, und gegebenenfalls eine Silicon-Antischaumemulsion enthält.

9. Arzneimittelzubereitung nach einem der Patentansprüche 1 oder 4-8, dadurch gekennzeichnet, dass die Membranschicht 45-55 Gew.-% Eudragit NE 30 D, 5-10 Gew.-% Povidone K 30, 0,5-1,5 Gew.-% Polysorbat 80, 0,5-1,5 Gew.-% Eisenoxid rot, 15-30 Gew.-% Talcum, 10-15 Gew.-% Mg-stearat, und gegebenenfalls 0,01-0,1 Gew.-%, jeweils bezogen auf die Membranschicht, Silicon-Antischaummittel enthält.

10. Arzneimittelzubereitung nach Patentanspruch 1, dadurch gekennzeichnet, dass die magensaftresistente Lackschicht 5-15 Gew.-%, bezogen auf die Endpellets, vorzugsweise 10 Gew.-%, beträgt und 60-90 Gew.-% der magensaftresistenten Lackschicht aus einem säureunlöslichen Polymer und 10-40 Gew.-%, jeweils bezogen auf die Lackschicht, aus Hilfsstoffen besteht.

**11.** Arzneimittelzubereitung nach Patentanspruch 1 oder 10, dadurch gekennzeichnet, dass die magensaftresistente Lackschicht ein Methacrylsäurepolymer, wie Eudragit L 30 D, Eudragit RL 30 D und/oder Eudragit RS 30 D, oder Cellulosederivate, wie Aquateric und/oder Duodcel, Weichmacher, wie Diethylphthalat, Triacetin, Dibutylsebacat, Diethyladipat, Polyethylenglykol und/oder Tributylcitrat, Gleitresp. Trennmittel, wie Talcum und/oder eine Kieselsäure, wie Syloid, gegebenenfalls Farbpigmente, wie Eisenoxid und/oder Titandioxid, und/oder gegebenenfalls ein Silicon-Antischaummittel enthält.

**12.** Arzneimittelzubereitung nach einem der Patentansprüche 1, 10 oder 11, dadurch gekennzeichnet, dass die magensaftresistente Lackschicht 5-15 Gew.-%, vorzugsweise 10 Gew.-%, bezogen auf die Endpellets ausmacht, und 70-80 Gew.-% Eudragit L 30 D, 10-20 Gew.-% Diethylphthalat, 5-15 Gew.-% Talcum und gegebenenfalls ein Silicon-Antischaummittel enthält.

**13.** Arzneimittelzubereitung nach einem der Patentansprüche 1-12, dadurch gekennzeichnet, dass das Neutralpellet 20-45 Gew.-% der Endpellets beträgt, die Wirkstoffschicht 20-45 Gew.-% Diclofenac und 5-15 Gew.-% Hilfsstoffe, jeweils bezogen auf die Endpellets, die Membranschicht 6-14 Gew.-% Eudragit NE 30 D (Trockenanteil), 0,5-4 Gew.-% Povidone K 30, 0,05-1 Gew.-% Polysorbat 80, 0,05-3 Gew.-% Eisenoxid rot, 1-6 Gew.-% Talcum, und 0,5-3 Gew.-% Mg-stearat, jeweils bezogen auf die Endpellets, und die magensaftresistente Lackschicht 5-12 Gew.-% Eudragit L 30 D (Trockenanteil) und 1-4 Gew.-%, jeweils bezogen auf die Endpellets, Diethylphthalat und Talcum enthält.

**14.** Arzneimittelzubereitung nach einem der Patentansprüche 1-13, dadurch gekennzeichnet, dass das Neutralpellet 31 Gew.-% der Endpellets beträgt, die Wirkstoffschicht 31 Gew.-% Diclofenac, 6 Gew.-% Povidone K 30, 1 Gew.-% Macrogol 400, 2 Gew.-% Talcum, und gegebenenfalls eine Silicon-Antischaumemulsion und 3 Gew.-% Nicotinamid, die Membranschicht 8 Gew.-% Eudragit NE 30 D (Trockenanteil), 0,2 Gew.-% Polysorbat 80, 1,5 Gew.-% Povidone K 30, 0,15 Gew.-% Eisenoxid rot, 3,5 Gew.-% Talcum, 2 Gew.-% Magnesiumstearat, und gegebenenfalls eine Silicon-Antischaumemulsion, und die Lackschicht 7,5 Gew.-% Eudragit L 30 D (Trockenanteil), 1,5 Gew.-% Diethylphthalat und 1 Gew.-%, jeweils bezogen auf die Endpellets, Talcum, und gegebenenfalls eine Silicon-Antischaumemulsion, enthält.

**15.** Eine Diclofenac oder eines seiner Salze enthaltende pelletierte orale Arzneimittelzubereitung mit gesteuerter Wirkstofffreisetzung, gekennzeichnet durch eine annähernde in-vitro-Freisetzungsrate von >15 % Wirkstoff nach 1 Stunde, >30 % nach 2 Stunden, >50 % nach 3 Stunden, >70 % nach 4 Stunden und >80 % nach 5 Stunden.

**16.** Kapsel, insbesondere Hartgelatinekapsel, enthaltend 75 mg Diclofenac oder eines seiner Salze in Form von Pellets gemäss einem der Patentansprüche 1-15.

**17.** Kapsel nach Patentanspruch 16, gekennzeichnet durch eine zweimalige Applikation pro Tag.

**18.** Verfahren zur Herstellung einer Diclofenac oder eines seiner Salze enthaltenden Arzneimittelzubereitung mit gesteuerter Wirkstofffreisetzung, dadurch gekennzeichnet, dass man in einem ersten Verfahrensschritt die Wirkstoffschicht durch Besprühen von Neutralpellets mittels einer den Wirkstoff enthaltenden Suspension bildet, in einem zweiten Verfahrensschritt die Membranschichtbildner auf die Wirkstoffschicht und in einem dritten Verfahrensschritt die suspendierten magensaftresistenten Lackschichtbildner auf die Membranschicht aufsprüht, die Pellets trocknet und dann diejenigen der geeigneten Korngrösse von >o,8 mm und <1,4 mm aussiebt.

**19.** Verfahren nach Patentanspruch 18, dadurch gekennzeichnet, dass das Verfahren in einer Wirbelschichtsprühanlage, die vorzugsweise einen Wurster-Einsatz aufweist, durchgeführt wird.

**20.** Verfahren nach Patentanspruch 18, dadurch gekennzeichnet, dass das Verfahren in einer Wirbelschichtsprühanlage, die einen Rotorprozessor-Einsatz aufweist, durchgeführt wird.

**21.** Verfahren nach einem der Patentansprüche 18-20, dadurch gekennzeichnet, dass man als Lösungsresp. Suspensionsmittel Wasser einsetzt.

FIG. 1

Freisetzung in vitro

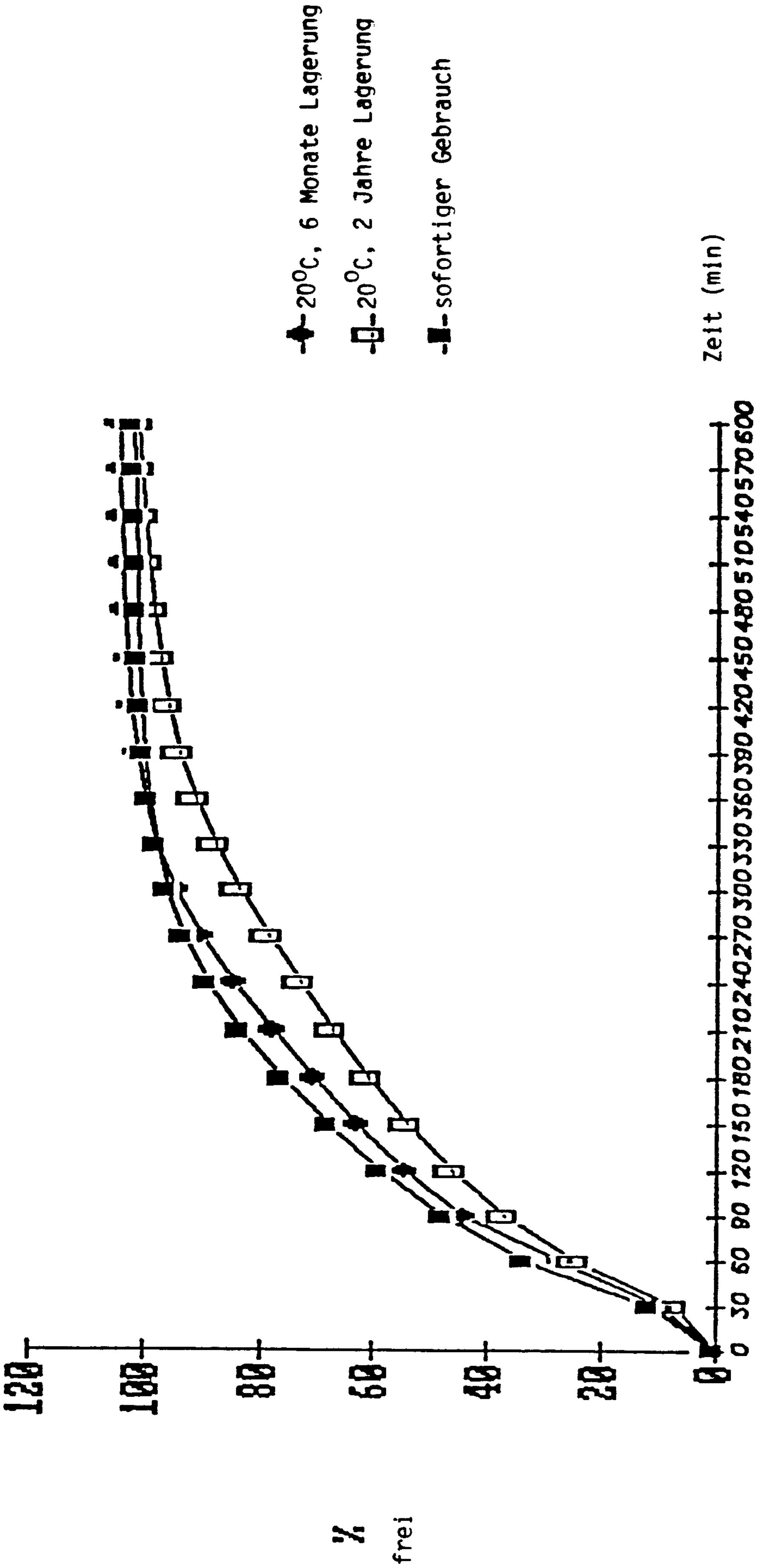

FIG. 2

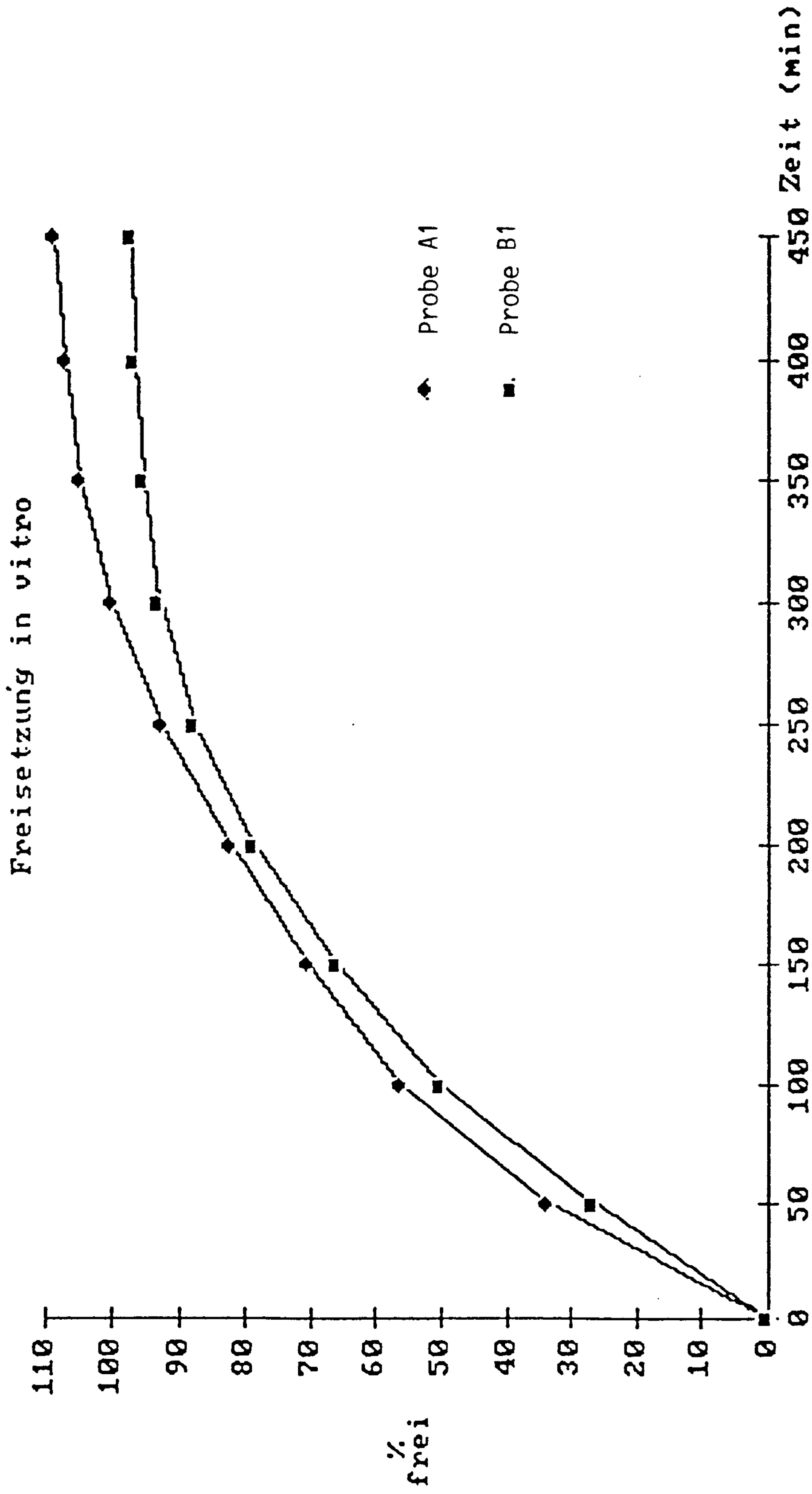

Freisetzung in vitro
% frei
110
100
90
80
70
60
50
40
30
20
10
0
0
50
100
150
200
250
300
350
400
450
Zeit (min)
Probe A1
Probe B1

FIG. 3

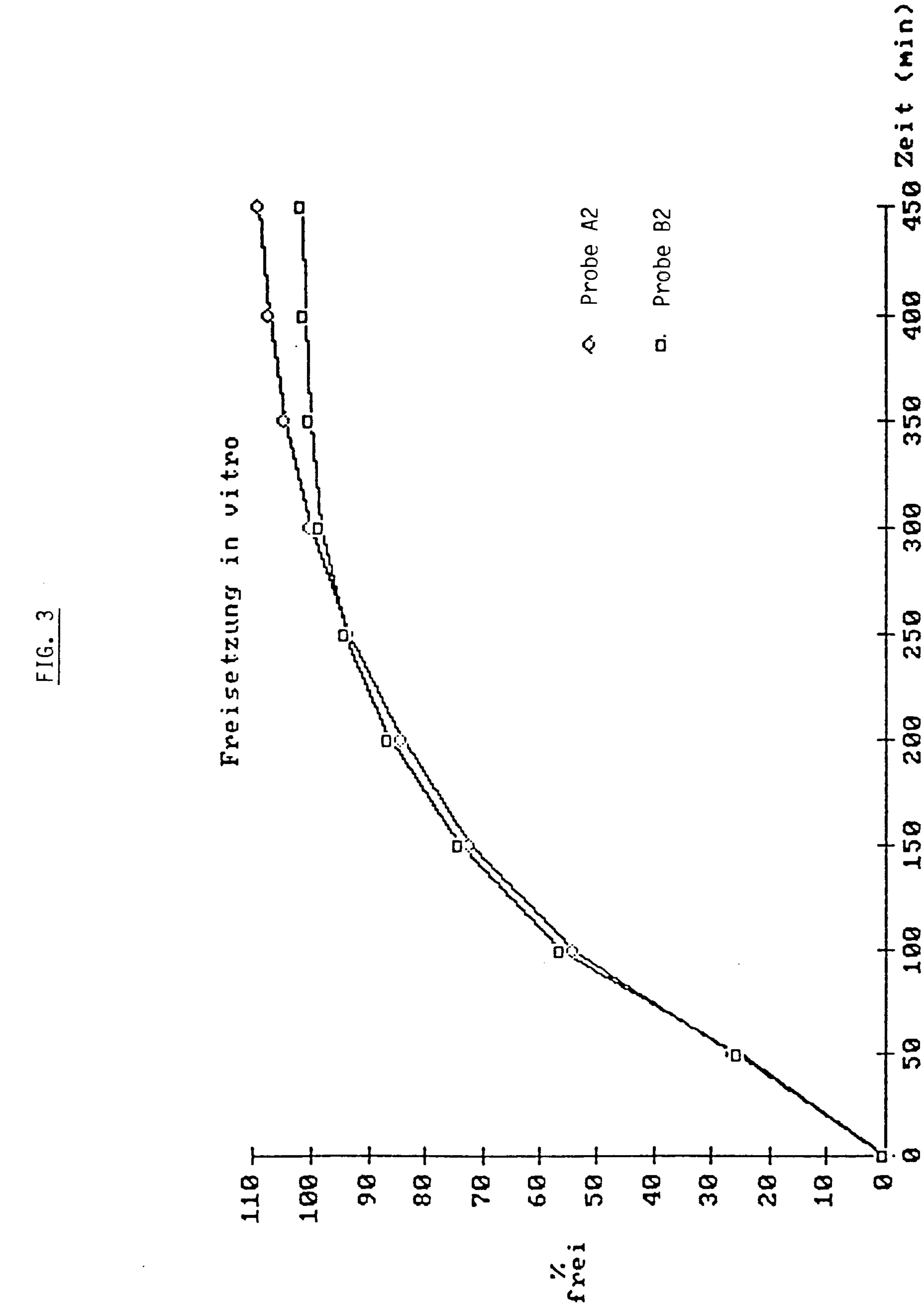
Freisetzung in vitro
% frei
110
100
90
80
70
60
50
40
30
20
10
0
0
50
100
150
200
250
300
350
400
450
Zeit (min)
◇ Probe A2
□ Probe B2

Europäisches Patentamt

# EUROPÄISCHER RECHERCHENBERICHT

Nummer der Anmeldung

EP 91 81 0460

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | FR-A-2 635 460 (SS PHARMACEUTICAL CO., LTD)<br>* Ansprüche 1,6; Seite 3, Zeile 14 - Seite 4, Zeile 5 *<br>--- | 1-18 | A 61 K 31/195<br>A 61 K 9/54 |
| A | EP-A-0 383 967 (DOJIN IYAKU-KAKO CO., LTD)<br>* Anspruch 1; Seite 3, Zeile 31 - Seite 4, Zeile 12 *<br>--- | 1-18 | |
| A | EP-A-0 421 921 (CIBA-GEIGY AG)<br>* Ansprüche 1,5,8; Seite 3, Zeile 13 - Seite 5, Zeile 6 *<br>----- | 1-18 | |
| | | | **RECHERCHIERTE SACHGEBIETE (Int. Cl.5)** |
| | | | A 61 K |

Der vorliegende Recherchenbericht wurde für alle Patentansprüche erstellt

| Recherchenort | Abschlußdatum der Recherche | Prüfer |
|---|---|---|
| DEN HAAG | 29-11-1991 | VENTURA AMAT A. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)